# EUROPEAN PATENT APPLICATION

(11) **EP 1 510 532 A1**
(43) Date of publication of application: **02.03.2005**
(21) Application number: 03077680.1
(22) Date of filing: 27.08.2003
(51) Int. Cl.: C08F 8/30, C08F 8/32, C07D 413/02, C08J 3/24

(54) **Process for the preparation of a functionalized polymer, intermediate products, processes for the preparation thereof, a functionalized polymer and polymer composition containing a functionalized polymer and shaped parts**

(71) Applicant: DSM IP Assets B.V., 6411 TE Heerlen (NL)
(72) Inventor: Loontjens, Jacobus Antonius, 6231 KK Meerssen (NL)
(74) Representative: Mooij, Johannes Jacobus

(57) **Abstract**

Process for the preparation of a functionalized polymer containing an additive, in which process there is first formed a compound that contains, besides at least one oxazine ring, a free amino or hydroxy group; this compound is linked to an additive via the free amino or hydroxy group in the additive; this additive linked to the compound mentioned is contacted with a polymer that contains at least one free carboxylic group, at a temperature above the polymer's melting point and at least above 150 °C, such that the oxazine ring reacts with the free carboxylic group of the polymer to form the functionalized polymer.

The invention also relates to the intermediate products formed in the process and in the preparation thereof.

Lastly, the invention relates to functionalized polymers and polymer compositions containing the functionalized polymer.

## Description

The invention relates to a process for the preparation of a functionalised polymer. The invention also relates to intermediate products, processes for the preparation thereof, a functionalized polymer and a polymer composition containing a functionalized polymer as well as shaped parts.

A functionalized polymer may be prepared by adding an additive to a polymer. Certain properties of the polymer are improved in this way. A stabilizer, for example, is added in order to improve the stability of a polymer. Additives blended in polymers generally tend to migrate out of the polymer. This is also known as the "bleeding" of additives. An additive that bleeds out of a polymer accumulates on the surface of the polymer. This means not only the loss of a valuable additive from the polymer, but there also develops a deposit on the polymer surface. Such a deposit on the surface of a polymer is undesirable for many applications. Furthermore, the additive, while migrating out of the polymer, may cause deposits in polymer processing equipment. In injection moulding of polymers, for example, the additive may deposit in a mould. As a consequence, the injection moulding operation needs to be interrupted in order to clean the mould. Such interruptions of production processes are undesirable from a cost viewpoint.

Furthermore, additives may bleed out of coatings. Additives that are added to a coating composition or paint may migrate to the surface. As a result, the performance of the additive present on the surface may be lost, which is undesirable. In a known method of preparing a functionalized polymer, the molecular weight of the additive is first increased and then the additive is blended in a polymer. This reduces the bleeding of additives out of polymers.

Another manner of preparing functionalized polymers is described by Wolfe in Rubber Chemistry and Technology (vol. 54, p. 988-995). Here, during the polymerization of segmented polyether ester elastomers, antioxidants are copolymerized by utilizing bifunctional monomers with antioxidant properties, in this application also referrred to as antioxidant. This prevents the antioxidant from bleeding out of the polymer. Dimethyl 5-(3,5-di-t-butyl-4-hydroxybenzenepropanamido)-isophthalate, among other substances, is mentioned as a bifunctional antioxidant.

A drawback of this method as described by Wolfe is that it can only be applied during the polymerization of the polymer.

The invention aims to provide a process for the preparation of functionalized polymers that is not limited to the polymerization of the polymer and that prevents the additive from migrating.

This is achieved according to the invention in that
a. a first compound, containing at least one primary amino group and at least a group chosen from a first series comprising a secondary amino group, an amino group on a secondary carbon atom and a primary hydroxyl group or a group chosen from a second series comprising a hydroxyl group on a secondary carbon atom, or a first compound containing at least one group chosen from the first series and also contains at least a group chosen from the second series whereby optionally said first or second series furthermore comprise a double or triple bond, is contacted with an amount of an oxazine compound according to formula (I), in which the cyclic structure is hereinafter referred to as oxazine ring. The oxazine compound according to formula (I) is hereinafter referred to as 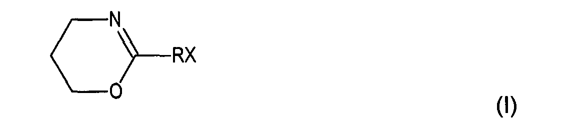 in which
   X = I, Br, Cl, OC(O)R₁, OS(O₂)R₂
   R, R₁, R₂ = an alkyl or aryl group, preferably with less than 10 carbon atoms, more preferably with less than 6 carbon atoms
   at a temperature below 150 °C and with the amount of oxazine being at least equimolar to the number of primary amino groups or at least equimolar to the number of groups chosen from the first series and with the molar amount of oxazine being lower than the sum of the molar number of primary amino groups and groups chosen from the first or second series or lower than the sum of the molar number of groups chosen from the first series and chosen from the second series, as a result of which a first intermediate compound is formed which contains, besides at least one oxazine ring, a free amino, hydroxy, or a double or triple bond;
b. the first intermediate compound is contacted, at a temperature preferably below 150 °C, with an additive such that a link is established via the free amino, hydroxy, or the double or triple bond, resulting in the formation of a second intermediate compound;
c. the second intermediate compound is contacted with a polymer having at least one free carboxyl group at a temperature above the melting point of the polymer and at least above 150 °C, such that the oxazine ring reacts with the free carboxylic group of the polymer to form the functionalized polymer.

The invention provides a process for the preparation of functionalized polymers, which process is not limited to the polymerization of this polymer and prevents an additive from bleeding. Large amounts of polymer are usually continuously produced during polymerization. This means that when an additive is added during the polymerization of the polymer, large amounts of a functionalized polymer,comprising said additive, are produced. The process according to the invention allows smaller batches of functionalised polymer to be produced more easily in, for example, a compounding process. By adding an additive not during polymerization but later, for example during compounding in an extruder or during injection moulding in an injection moulding machine, greater flexibility is obtained than during the production of a polymer or a polymer composition. In addition, the continuous polymerization process is not disturbed in this manner.

In the process according to the invention a first reaction is carried out as mentioned under a, above. By way of an example of a first reaction, a reaction A) is shown below, in which a first compound, I, is ethanol amine which is reacted with 2-chloromethyl-2-oxazine. In reaction equation A), the first compound reacts with two moles of 2-chloromethyl-2-oxazine to form the first intermediate compound, II, with two moles of hydrochloric acid being split off.

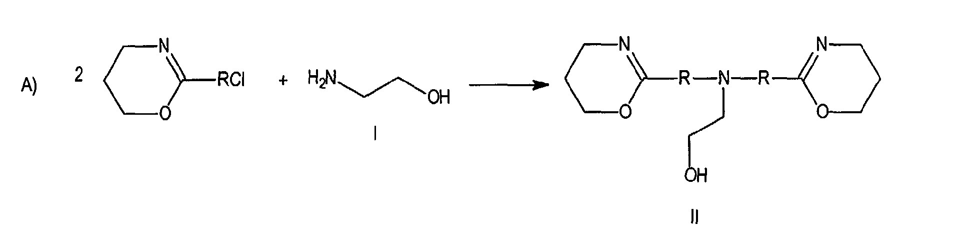

The reaction may be carried out 'in bulk', with the first compound and the oxazine being contacted directly, but the reaction may also be carried out in solution.

A base can optionally be added to scavenge the HCl, that is eliminated during this reaction.

Examples of bases, which include Lewis bases, that are suitable as an acid scavenger are MHₙ, M(OH)ₙ, (R'O)ₙM (M= Alkali or earth alkali, R' = alkyl with C₁ - C₂₀ or aryl), NR'ₙH₄₋ₙOH (R' = alkyl with C₁ - C₂₀ or aryl), triamines such as triethylamine, tributyl amine, trihexylamine, trioctylamine and cyclic amines such as diazobicyclo[2,2,2]octane (DABCO), dimethylaminopyridine (DMAP), guanidine, morfoline.

If a solvent is used, preferably an aprotic solvent is used. This prevents unwanted reactions with the solvent. Suitable aprotic solvents are for example aliphatic or aromatic hydrocarbons such as toluene or xylene.

The reaction of step a. of the invention is carried out at a temperature below 150 °C. Undesirable side reactions may take place above 150 °C, potentially resulting in a compound with less or no oxazine anymore. The reaction is preferably carried out at a temperature below 125 °C. If the boiling point of the chosen solvent is lower than the desired reaction temperature, the reaction may, if desired, be carried out under pressure and/or reflux. In general the reaction is carried out at a temperature above room temperature, preferably above 50 °C. Long reaction times are prevented in this manner. A suitable first compound is one containing at least one primary amino group and at least a group chosen from a first series comprising a secondary amino group, an amino group on a secondary carbon atom, a primary hydroxyl group or a group chosen from a second series comprising a hydroxyl group on a secondary carbon atom, or a first compound containing at least one group chosen from the first series and also at least one group chosen from the second series. The chosen groups may be mutually bonded by one or more aliphatic, cycloaliphatic or aromatic units chosen independently of one another.

Examples of suitable first compounds are for example bishexamethylenetriamine, bisethylenetriamine, bispropylenetriamine, 2-(ethylamino)ethylamine, 3-(methylamino)propylamine, 3-(cylohexylamino)propylamine, 1,2-propanediamine, N,N'-1,2-ethanediylbis-(1,3-propanediamine), N-(aminoethyl)benzylamine. Examples of a first compound containing amino and hydroxy groups are for example ethanolamine, propanolamine, isopropanolamine, 2-(2-aminoethyoxy)ethanol, N-(2-aminoethyl)ethanolamine, N-methylethanolamine, diethanolamine, chitin. Further examples of a first compound comprising a primary hydroxy group and a hydroxy group on a secondary carbon atom are glycerol, 1,2-pentanediol, 1,2,4-butanetriol or glucose. Examples of first compounds containing amino and carboxy groups are glycine, asparagine, lysine, glutamine or y-aminocapronic acid.

Various types may be used as oxazine. Preferably 2-chloromethyl-2-oxazine, is used as this is easily produced from monochloric acetic acid and propanolamine.

In the reaction of the invention referred to under b., the first intermediate compound is contacted, preferably at a temperature below 150 °C, with an additive such that a link is established via the free amino, hydroxy or via a double or triple bond to form a second intermediate compound. The additive's linkage to the first intermediate compound takes place via a reactive group that is present on the additive. An example of this link is shown in the reaction equation B) below, which is based on the reaction product of reaction A) and wherein YFn symbolizes the additive containing a reactive group Y. If the first intermediate compound contains several reactive groups per molecule, then several molecules of the additive per molecule of the first intermediate compound may be linked.

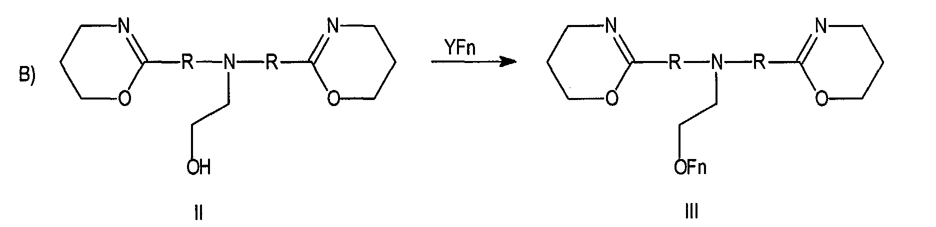

The linkage may take place if the additive contains a reactive group Y capable of reacting directly with a group of the first intermediate compound. Reactive groups on the additive may be a halide, an ester, an isocyanate, an epoxy, an aldehyde or an anhydride. In some cases the linkage cannot take place directly, for example because both the first intermediate compound and the additive contain groups that do not react directly with one another, for example when both contain a hydroxyl, or amino group. In such cases the linkage may take place via a so-called linking unit. This linking unit contains one reactive group capable of reacting with the reactive group of the first intermediate compound and one reactive group capable of reacting with the reactive group present in the additive. If the linking unit is to link with a hydroxyl group, the linking unit preferably contains an acid group, an isocyanate, a dihalogenide or a cyclic anhydride. If the linking unit is to link with an amino group, it preferably contains an acid group, an isocyanate, an aldehyde or a cyclic anhydride, or carbonylbislactam. If the linking unit is to link with a carboxy group, it preferably contains an amino group or hydroxy group. Suitable linking units may be cyclic anhydrides, diisocyanates or aldehydes.

Examples of cyclic anhydrides are for example succinic anhydride, maleic anhydride or phthalic anhydride. A diisocyanate that may be suitable is for example isophorondiisocyanate (IPDI) or toluenediisocyanate (TDI). Examples of aldehydes are for example formaldehyde, acetaldehyde, benzaldehyde or glyoxal. If a linking unit is used, it is preferably first reacted with the first intermediate compound or the additive and the formed product is subsequently, in a next reaction step, reacted with the additive or the first intermediate compound respectively.

An additive in this invention is understood to be an antioxidant, a flame retardant, a bactericide, a fungicide, a dying agent, a surfactant, an anti-fouling agent, a colouring agent, an antistatic agent or a lubricant, or a combination thereof.

The reaction is preferably carried out at a temperature below 150 °C, because otherwise undesirable side reactions may take place. In general the reaction is carried out at a temperature above room temperature, preferably above 50 °C. Long reaction times are prevented in this manner.

In the reaction referred to under c. above, the second intermediate compound is contacted with a polymer having at least one free carboxylic group such that the oxazine group of the second intermediate compound reacts with the free carboxylic group of the polymer. In the process according to the invention the second intermediate compound is preferably added to the polymer in an extruder or injection moulding machine. In this manner, the second intermediate compound is rapidly blended with the polymer and a quick reaction takes place at the high temperatures during extrusion or injection moulding. Under these conditions the reaction will usually be complete in a few minutes. The second intermediate compound may also be added during the production of coating compositions. The second intermediate compound may be metered directly to the extruder or may be added to the extruder or injection moulding machine pre-blended with the polymer or other additives. If the second intermediate compound is liquid, it may also be added to the extruder or injection moulding machine with a liquid metering system. It is possible to add additives that react with hydroy or amine froups if present, such as carbonyl biscaprolactam, diisocyanates, etc..

If the second intermediate compound contains one oxazine group, the second intermediate compound may be linked to the end of the polymer chain. If the second intermediate compound contains several oxazine groups, it may also be incorporated in the polymer. If the second intermediate compound links two polymer chains, chain extension may occur. This is manifested by an increase in the molar mass of the polymer. An extra advantage of the invention is that additives having only one functional group may also be used. In the publication by Wolfe cited above this is not possible since the addition of a monofunctional compound during the polymerization may result in chain termination, as a result of which the polymer's molecular mass is limited. Also, the addition, via the process of the invention, of the additives cited by Wolfe will not result in a functionalized polymer in which additives do not bleed, since in general the additives cited by Wolfe react only slowly.

Consequently, over the relatively short reaction time in an extruder, practically no reaction takes place with the polymer, so allowing the additives still to bleed out of the polymer.

The second intermediate compound is contacted with the polymer containing at least one free carboxylic group at a temperature above the melting point of the polymer and at least at 150 °C. At lower temperatures the reaction will proceed more slowly and will not run to completion during the residence time in the extruder. The upper temperature limit is not subject to any further limitation than the temperature customary for melt processing of the polymers in question.

Polymers that contain a free carboxylic group are for example polyamides, polyesters, copolyesters, polyethers, polyacrylates, and carboxylic functionalized polymers or copolymers or blends thereof.

Exemplary polyamides are polyamides and copolyamides that are derived from diamines and dicarboxylic acids and/or from aminocarboxylic acids or the corresponding lactams, such as polyamide 4, polyamide 6, polyamide 11, polyamide 12, polyamide 6/6, 4/6, partially aromatic (co)polyamides, for example polyamides based on an aromatic diamine and adipic acid; polyamides produced from an alkylenediamine and isophthalic and/or terephthalic acid and copolyamides thereof.

Exemplary polyesters are polyesters derived from dicarboxylic acids as e.g terephthalic acid, isophthalic acid and trimellitic acid,and dialcohols as e.g. ethylene glycol, propane diol, butanediol, neopentyl glycol and/or from hydroxycarboxylic acids or the corresponding lactones including polyethyleneterephthalate, polypropyleneterephthalate, polybutyleneterephthalate, poly-1,4-dimethylolcyclohexaneterephthalate, polycaprolacton and copolyesters thereof or thermosetting polyesters derived of any one of the above mentioned monomers.

Examples of amine functionalized polymers are for example carboxylic functionalized polyethers including carboxylic terminated acrylonitrilebutadiene copolymers (CTBN).

The amount of the second intermediate compound to be added to the polymer may be freely chosen. Preferably the amount of the additive is chosen to be equal to or lower than the amount of free carboxylic groups. These groups may be determined by techniques known to those skilled in the art. In general, a polymer with a lower molar mass contains more free carboxylic groups so that a larger amount of the second intermediate compound may be linked to a polymer with a lower molar mass. The amount of the second intermediate compound is preferably so chosen that the amount of additive to be introduced in the polymer is equal to the desired dosage of the additive in the polymer composition. If a larger amount of additive is to be added to the polymer via the second intermediate compound than the available terminal groups in this polymer, this may be accomplished by using a second intermediate compound to each molecule of which more than one molecule of the additive is linked.

If desired, fillers may be added during compounding. In general, there may be added glass fibres, glass spheres, glass lamellae, mineral fillers including for example, mica, talcum, chalk or gypsum, rubbers, including for example halogenated polymer systems and synergists such as antimonetrioxide, sodiumantimonate or zinc borate.

The invention also relates to a first intermediate compound comprising at least one oxazine group and a free amino or hydroxy group.

The invention further relates to a second intermediate compound comprising an additive that is linked to a compound containing at least one oxazine group. In this second intermediate compound the additive may be linked to the first intermediate compound via the free amino, hydroxy group or via a double or triple bond present in the first intermediate compound.

Furthermore, the invention relates to a process for obtaining the said intermediate compounds and product. It concerns a process for the preparation of the first intermediate compound, wherein the process comprises step a. of the process of the invention.

The invention further relates to a process for the preparation of the second intermediate compound wherein the process comprises steps mentioned under
a. and b. of the process of the invention. In this process the additive is chosen from the series of stabilizers, flame retardants, bactericides, fungicides, dying agents, surfactants, anti-fouling agents, colouring agents, antistatic agents and lubricants. The invention further relates to an alternative route to produce a functionalised polymer using an additive comprising an oxazine groups. Such process comprises

a. reacting an additive comprising at least one amino group or a hydroxyl group with said oxazine at a temperature below 150 °C such that a link is established via the amino group or hydroxyl group of the additive, thereby forming an intermediate product A,
b. contacting the intermediate product A with a polymer having at least one free carboxylic group at a temperature above the melting point of the polymer and at least above 150 °C, such that the oxazine ring reacts with the free carboxylic group of the polymer to form a functionalized polymer.

In this process an intermediate product A is formed. The invention therefore also relates to an intermediate product A comprising an additive provided with an oxazine group as well as the process for the preparation of an intermediate product A, which comprises step a. of the above mentioned alternative process according to the invention.

The invention also provides a functionalised polymer obtainable by the process of the invention.

Such a functionalized polymer may also be added to other polymers, this group of other polymers not necessarily being limited to the group of polymers that contain a free carboxylic group. The invention therefore also provides polymer compositions containing also a functionalized polymer with an additive that does not bleed.

Said functionalized polymers or polymer compositions according to the invention can also be applied in coating compositions. The invention therefore also provides a coating composition comprising functionalized polymers according to the invention or shaped articles comprising a functionalised polymer composition according to the invention. The coating composition can further comprise the usual fillers applied in coating chemistry.

Upon curing of the coating composition according to the invention a coating is obtained on a substrate. The invention therefore furthermore provides substrates comprising a coating based on the coating composition according to the invention.

Furthermore the invention relates to shaped articles comprising functionalized polymers according to the invention or shaped articles comprising functionalised polymer compositions according to the invention. The shaped articles can be produced by the known methods as e.g. injection moulding or extrusion. Consequently shaped articles comprise moulded parts extruded parts, films, strapping and fibres.

## Claims

1. Process for the preparation of a functionalized polymer **characterized in that**
a. a first compound, containing at least a primary amino group and at least a group chosen from a first series comprising a secondary amino group, an amino group attached to a secondary carbon atom and a primary hydroxyl group or a group chosen from a second series comprising a hydroxyl group attached to a secondary carbon atom, or a first compound containing at least a group chosen from the first series and also contains at least a group chosen from the second series whereby optionally said first or second series furthermore comprise a double or triple bond, is contacted with an amount of oxazine compound according to formula (I) 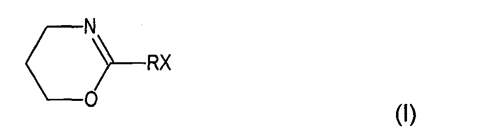 in which
X = I, Br, Cl, OC(O)R₁, OS(O₂)R₂
R, R₁, R₂ = an alkyl or aryl group, preferably with less than 10 carbon atoms, more preferably with less than 6 carbon atoms
at a temperature below 150 °C and with the amount of oxazine being at least equimolar to the number of primary amino groups or at least equimolar to the number of groups chosen from the first series and with the molar amount of oxazine being lower than the sum of the molar number of primary amino groups and groups chosen from the first or second series or lower than the sum of the molar number of groups chosen from the first series and chosen from the second series, as a result of which a first intermediate compound is formed which contains, besides at least one oxazine ring, a free amino group, hydroxy group, or a double or triple bond;
b. the first intermediate compound is contacted, at a temperature preferably below 150 °C, with an additive such that a link is established via the free amino, hydroxy or via a double or triple bond to form a second intermediate compound.
c. the second intermediate compound is contacted with a polymer having at least one carboxylic group at a temperature above the melting point of the polymer and at least above 150 °C, such that the oxazine ring reacts with the free carboxylic group of the polymer to form the functionalized polymer.

2. Process according to Claim 1 wherein said oxazine is 2-chloromethyl-2-oxazine.

3. Process according to Claim 1 or 2 wherein the polymer is chosen from the series of polyamides, polyesters, copolyesters, polyethers and polyacrylates.

4. First intermediate compound comprising at least one oxazine ring and a free amino or hydroxy group, or a double or triple bond.

5. Process for the preparation of the compound of Claim 4, **characterized in that** the process comprises a step as mentioned under a. in Claim 1.

6. Second intermediate compound comprising an additive that is linked to the first intermediate compound according to Claim 4 via the free amino or hydroxy group or a double or triple bond present in the first intermediate compound.

7. Second intermediate compound according to Claim 6 wherein the additive is chosen from the series of stabilizers, flame retardants, bactericides, fungicides, surfactants, anti-fouling agents, colouring agents, antistatic agents and lubricants.

8. Process for the preparation of the second intermediate compound according to Claim 6 or 7, **characterized in that** the process comprises steps as mentioned under a. and b. in Claim 1.

9. Process for the preparation of a functionalized polymer by
a. reacting an additive comprising at least one amino group or a hydroxyl group with the oxazine compound according to formula (I) at a temperature below 150 °C such that a link is established via the amino group or hydroxyl group of the additive, thereby forming an intermediate product A,
b. contacting the intermediate product A with a polymer having at least one free carboxylic group at a temperature above the melting point of the polymer and at least above 150 °C, such that the oxazine ring reacts with the free carboxylic group of the polymer to form a functionalized polymer.

10. Intermediate product A comprising an additive provided with an oxazine ring.

11. Process for the preparation of an intermediate product A according to Claim 10, **characterized in that** the process comprises a step as mentioned under a. in Claim 9.

12. Functionalized polymer obtainable according to the process of any one of Claims 1-3 or 9.

13. Polymer composition containing a functionalized polymer according to Claim 12.

14. Shaped article comprising the polymer composition of claim 13.

15. Shaped article according to claim 14 wherein the shaped article is a film, fibre, monofilament or strapping.

16. Coating composition comprising the second intermediate compound of any one of claims 6-7.

17. Substrate comprising a coating based on the coating composition according to claim 15.
